# EUROPÄISCHE PATENTSCHRIFT

(11) **EP 1 003 490 B1**
(45) Veröffentlichungstag und Bekanntmachung des Hinweises auf die Patenterteilung: **11.09.2002**
(21) Anmeldenummer: 98939550.4
(22) Anmeldetag: 29.06.1998
(51) Int. Cl.: A61K 9/70, A61K 31/56

(54) **TRANSDERMALES THERAPEUTISCHES SYSTEM (TTS) ZUR VERABREICHUNG VON SEXUALSTEROIDHORMONEN**
TRANSDERMAL THERAPEUTIC SYSTEM (TTS) FOR ADMINISTERING SEXUAL STEROID HORMONES
SYSTEME THERAPEUTIQUE TRANSDERMIQUE (TTS) POUR ADMINISTRER DES HORMONES STEROIDES SEXUELLES

(30) Priorität: 04.07.1997 DE 19728517
(43) Veröffentlichungstag der Anmeldung: 31.05.2000
(73) Patentinhaber: SCHWARZ PHARMA AG, 40789 Monheim (DE)
(72) Erfinder: WOLFF, Hans-Michael, D-40789 Monheim (DE); ARTH, Christoph, D-40593 Düsseldorf (DE)
(86) Internationale Anmeldenummer: EP9803950
(87) Internationale Veröffentlichungsnummer: WO99001116

(56) Entgegenhaltungen:
- EP-A- 0 563 507
- EP-A- 0 848 960
- DE-A- 4 310 012

## Beschreibung

Die vorliegende Erfindung betrifft ein Transdermales Therapeutisches System (TTS) zur Verabreichung von Sexualsteroidhormonen durch die Haut über längere Zeiträume, sowie ein Verfahren zu seiner Herstellung ohne Verwendung von Lösungsmitteln, das besonders wirkstoffschonend ist.

Die Bioverfügbarkeit von oral verabreichten Wirkstoffen ist oft unbefriedigend. Die hepatische Metabolisierung vieler Wirkstoffe kann bei der ersten Leberpassage zu unerwünschten Konzentrationsverhältnissen, toxischen Nebenprodukten und zur Verminderung der Wirkung oder gar zum Wirkverlust führen. Gegenüber oraler Verabreichung besitzt die transdermale Gabe von Wirkstoffen verschiedene Vorteile. Die Wirkstoffzufuhr läßt sich über einen längeren Zeitraum besser steuern, wodurch hohe Blutspiegelschwankungen vermieden werden. Zudem kann die erforderliche therapeutisch wirksame Dosis meist deutlich verringert werden. Außerdem wird ein Pflaster vom Patienten oft mehr bevorzugt als täglich einmal oder mehrfach einzunehmende Tabletten.

In der Vergangenheit wurde zur Überwindung der vorgenannten Nachteile der nichttransdermalen Gabe von Wirkstoffen durch eine Vielzahl von Transdermalen Therapeutischen Systemen (TTS) mit unterschiedlichem Aufbau für verschiedene Wirkstoffe zur Therapie unterschiedlicher Erkrankungen Rechnung getragen.

So beschreiben die nachfolgend genannten technischen Dokumente für eine breite Vielfalt systemisch oder lokal reagierender Wirkstoffe deren parenterale Verabreichung entweder auf Basis dosis-kontrollierender oder allgemein freisetzender Systeme.

Beispielhaft sind dies: U.S.P. 3,598,122; 3,598,123; 3,731,683; 3,797,494; 4,031,894; 4,201,211; 4,286,592; 4,314,557; 4,379,454; 4,435,180; 4,559,222; 4,568,343; 4,573,995, 4,588,580; 4,645,502; 4,702,282; 4,788,062; 4,816,258; 4,849,226; 4,908,027; 4,943,435 und 5,004,610.

In den späten sechziger Jahren dieses Jahrhunderts war ursprünglich theoretisch angenommen worden, daß jeder Wirkstoff mit kurzer Halbwertszeit aber hoher Wirksamkeit und guter Hautdurchgängigkeit für eine sichere und effektive Verabreichung mittels eines TTS geeignet sei, Diese anfänglichen Erwartungen hinsichtlich der Möglichkeiten der transdermalen Verabreichung von Wirkstoffen mittels TTS konnten jedoch nicht erfüllt werden. Dies findet seine Begründung hauptsächlich darin, daß die Haut von Natur aus mit einer unüberschaubaren Vielfalt von Eigenschaften ausgestattet ist, um ihre Funktion als intakte Barriere gegenüber dem Eindringen von nicht-körpereigenen Substanzen in den Körper aufrecht zu erhalten. (Siehe hierzu: Transdermal Drug Delivery: Problems and Possibilities, B.M. Knepp et al., CRC Critical Review and Therapeutic Drug Carrier Systems, Vol. 4, Issue 1 (1987)).

Daher steht die transdermale Verabreichung nur für diejenigen wenigen Wirkstoffe zur Verfügung, die eine geeignete Kombination von vielen günstigen Charakteristika aufweisen. Für einen bestimmten Wirkstoff sind alle geforderten Charakteristika, die die sichere und effektive transdermale Verabreichung gewährleisten, weder theoretisch noch praktisch vorhersagbar.

Die an einen für die transdermale Verabreichung geeigneten Wirkstoff zu stellenden Anforderungen sind:
- Hautdurchgängigkeit,
- keine Beeinträchtigung des Klebevermögens des Pflasters durch den Wirkstoff,
- Vermeidung von Hautirritationen,
- Vermeidung von allergischen Reaktionen,
- günstige pharmakokinetische Eigenschaften,
- günstige pharmakodynamische Eigenschaften,
- ein relativ weites therapeutisches Fenster,
- Metabolismuseigenschaften, die konsistent mit der therapeutischen Anwendung bei kontinuierlicher Gabe sind.

Unzweifelhaft ist die vorgenannte Liste der Anforderungen nicht erschöpfend. Damit ein Wirkstoff für die transdermale Verabreichung zur Verfügung stehen kann, ist die "richtige" Kombination all dieser Anforderungen wünschenswert.

Das für die Wirkstoffe vorgenannte gilt in gleicher Weise für die den jeweiligen Wirkstoff enthaltende TTS-Zusammensetzung und deren konstruktiven Aufbau.

Üblicherweise handelt es sich bei den Transdermalen Therapeutischen Systemen (TTS) um Pflaster, die mit einer undurchlässigen Deckschicht, einer abziehbaren Schutzschicht und einer wirkstoffhaltigen Matrix oder einem wirkstoffhaltigen Reservoir mit semipermeabler Membran ausgestattet sind. Im ersten Fall werden sie als Matrixpflaster, im zweiten Fall als Membransystem bezeichnet.

Für die Deckschicht werden üblicherweise Folien aus Polyester, Polypropylen, Polyethylen, Polyurethan etc. verwendet, die auch metallisiert oder pigmentiert sein können. Für die abziehbare Schutzschicht kommen u.a. Folien aus Polyester, Polypropylen oder auch Papier mit Silikon- und/oder Polyethylenbeschichtung in Betracht.

Für die pharmazeutisch bzw. medizinisch üblichen wirkstoffhaltigen Matrices werden Polymermaterialien auf Basis von Polyacrylat, Silikon, Polyisobutylen, Butylkautschuk, Styrol/Butadien-Copolymerisat oder Styrol/lsopren-Copolymerisat verwendet.

Die in Membransystemen verwendeten Membranen können mikroporös oder semipermeabel sein und werden üblicherweise auf Basis eines inerten Polymeren, insbesondere Polypropylen, Polyvinylacetat oder Silikon gebildet.

Während die wirkstoffhaltigen Matrixzusammensetzungen selbstklebend sein können, ergeben sich aber auch in Abhängigkeit von eingesetztem Wirkstoff wirkstoffhaltige Matrices, die nicht selbstklebend sind, so daß als Folge hiervon das Pflaster oder TTS konstruktiv mit einem Overtape versehen werden muß.

Zur Sicherstellung der erforderlichen Fluxrate des Wirkstoffes sind häufig Hautpenetrationsenhancer wie aliphatische, cycloaliphatische und/oder aromatisch-aliphatische Alkohole, jeweils ein- oder mehrwertig und jeweils mit bis zu 8 C-Atomen umfassend, ein Alkohol-/Wasser-Gemisch, ein gesättigter und/oder ungesättigter Fettalkohol mit jeweils 8 bis 18 Kohlenstoffatomen, eine gesättigte und/oder ungesättigte Fettsäure mit jeweils 8 bis 18 Kohlenstoffatomen und/oder deren Ester sowie Vitamine als Zusatz erforderlich.

Weiterhin werden häufig Stabilisatoren, wie Polyvinylpyrrolidon, α-Tocopherolsuccinat, Propylgallat, Methionin, Cystein und/oder Cystein-hydrochlorid, der wirkstoffhaltigen Matrix zugesetzt.

Wie die vorgenannte Aufstellung zeigt, sind zahlreiche TTS-Konstruktionen und hierfür verwendete Materialien bekannt. Allerdings sind viele interagierende Erfordernisse zu berücksichtigen, wenn ein Medikament in Form eines TTS einem medizinischen Bedürfnis genügen soll.

Die folgenden Problemstellungen sind bei der Entwicklung von wirkstoffhaltigen TTS grundsätzlich zu berücksichtigen:
1. Zum Erreichen therapeutisch notwendiger Penetrationsraten des Wirkstoffes durch die Haut ist meist eine hohe Wirkstoffbeladung der Polymermatrix erforderlich. Nach Applikationsende im TTS verbleibender Wirkstoff wird therapeutisch ungenutzt mit dem Pflaster entsorgt. Dies ist jedoch, insbesondere bei hochwirksamen und teuren Wirkstoffen, aus Umweltschutz- und Kostengründen unerwünscht.
2. Die wirkstoffbeladene und ggf. zusätzlich mit Hautpenetrationsenhancern beladene Polymermatrix ist bei längerer Lagerung physikalisch nicht stabil. Insbesondere kann eine Wirkstoffrekristallisation auftreten, die zu einer nicht kontrollierbaren Abnahme der Wirkstofffreisetzungskapazität des TTS führt.
3. Eine hohe Beladung des polymeren Trägerstoffes mit Wirkstoff und/oder Hautpenetrationsenhancern erschwert bei selbstklebenden Polymerfilmen die Einstellung optimaler Hafteigenschaften des transdermalen Systems.
4. Die Wirkstoffresorptionsrate sinkt bei Anwendungen über mehrere Tage in nicht akzeptabler Weise ab, so daß zusätzliche Steuerschichten und/oder -komponenten erforderlich sind.
5. Werden wirkstoffbeladene Schichten aus organischen Lösungen hergestellt, tritt das Problem des Verbleibens von Lösemittelresten in der wirkstoffhaltigen Schicht nach dem Trocknungsprozeß auf. Zusätzlich besteht die Gefahr einer unerwünschten Verdunstung von flüchtigen Hilfsstoffen während der Herstellung. Da aus Gründen der physikalischen Stabilität und Hautverträglichkeit des Systems in der Regel vollständige Lösungsmittelfreiheit anzustreben ist, muß das Reservoir gegebenenfalls in mehreren Schichten aufgebaut werden. Dies wiederum führt zu einer Erhöhung der Herstellungskosten.

Die beschriebenen Probleme bedingen daher eine Vielzahl von Ausführungsformen Transdermaler Therapeutischer Systeme, die sich im Stand der Technik auf diesem Gebiet widerspiegeln.

Eine neuere Übersicht hierzu gibt beispielsweise **U.S. P 5,662,926** (Wick et al., 1997). Dieses Dokument beschreibt transdermale Systeme, die einen monolithischen, thermoplastischen Polymerfilm enthalten, in dem ein Wirkstoff, vorzugsweise Nikotin, homogen verteilt ist, sowie ein Verfahren zur lösungsmittelfreien Herstellung dieser wirkstoffhaltigen Schicht durch Mischen des wirksamen Bestandteils mit dem polymeren Trägermaterial in der Polymerschmelze bei Temperaturen von 170°C bis 200°C. Zur Fixierung des wirkstoffhaltigen Matrixfilms auf der Haut dient ein zusätzlicher Kontaktkleberfilm, der auf die Wirkstoffmatrix aufgebracht wird und, falls erforderlich, zusätzlich ein flächengrößeres Pflaster, das auf der von der Haut abgewandten Matrixseite auf den wirkstoffhaltigen Polymerfilm aufgebracht wird.

Besondere pharmazeutisch technische Probleme sind bei der Entwicklung von Östrogenpflastern zu lösen, die zur Behandlung klimakterischer Beschwerden appliziert werden müssen. Die Applikation sollte nur ein- oder zweimal wöchentlich erfolgen. Zunehmend Beachtung finden dabei sog. 7-Tage-Pflaster aus Kosten- und Patientencompliancegründen in dieser Indikation. Kostenaspekte spielen hier insofern eine besondere Bedeutung, als viele Sexualsteroidhormone hochpreisige Arzneistoffe darstellen, die für eine Dauertherapie vorgesehen sind. Zudem ist bei der Hormonverabreichung aus medizinischen Gründen nicht selten eine Kombinationstherapie erwünscht. So wird das natürliche Östrogen - 17β-Estradiol - zur Behandlung von klimakterischen Beschwerden in der Regel entweder kontinuierlich oder sequentiell zusammen mit einem Gestagen verabreicht.

Eine hinlänglich bekannte Ausführungsform solcher TTS stellen monolithische Wirkstoffpflaster dar, die eine kontrollierte Abgabe der wirksamen Bestandteile aus einer dünnen Haftklebeschicht ermöglichen. In der Praxis stößt jedoch die Entwicklung solcher Wirkstoffpflaster mit Sexualsteroidhormonen, insbesondere bei einer Anwendung über mehrere Tage, auf eine oder mehrere der nachfolgend genannten Schwierigkeiten, die häufig nur durch aufwendige Maßnahmen umgangen werden können und die Entwicklungs- und/oder Herstellkosten erhöhen. Es handelt sich im wesentlichen um folgende Problemstellungen:
1. Das Sexualsteroidhormon wird aus den Haftkleberfilmen in einer relativ niedrigen Rate pro Zeiteinheit durch die Haut freigesetzt, mit der Folge, daß relativ großflächige Pflaster appliziert werden müssen, um therapeutisch notwendige Hormonspiegel im Blut über längere Zeit aufzubauen und/oder daß sog. Penetrationsbeschleuniger zusammen mit dem oder den wirksamen Bestandteilen verabreicht werden müssen, um die erforderliche transepidermale Transportrate zu erzielen.
2. Das Sexualsteroidhormon ist in dem selbstklebenden Film in Abhängigkeit von den Aufbewahrungsbedingungen physikalisch instabil, d.h. es besteht insbesondere die Gefahr einer Wirkstoffrekristallisation bei Lagerung, die verbunden ist mit einer nicht kontrollierbaren Abnahme der Wirkstofffreisetzungskapazität.
3. Die Wirkstoffresorptionsrate sinkt bei Anwendung über mehrere Tage in nicht akzeptabler Weise, so daß zusätzliche Steuerschichten oder -komponenten erforderlich sind.
4. Eine hohe Beladung mit Wirkstoff und Penetrationsbeschleunigern erschwert in der Entwicklung die Einstellung optimaler Hafteigenschaften des TTS.
   Besondere Probleme stellen z.B. ein kalter Fluß der selbstklebenden Reservoirschicht dar, der bei der Humananwendung zu einem Austreten von wirkstoffhaltiger Masse über den Pflasterrand hinaus und damit zu Schmutzrändern führen kann. Weiterhin ist teilweises oder vollständiges Ablösen des TTS, bedingt durch Feuchtigkeitseinwirkung (z.B. beim Duschen, Schwimmen, starkem Schwitzen) und/oder durch starke Scherbeanspruchungen infolge von Muskel- resp. Hautbewegungen an der Grenzfläche Haut/Pflaster festzustellen.
5. Reservoirschichten für die transdermale Applikation werden häufig aus Lösungen hergestellt, so daß das Problem des Verbleibens von Lösemittelresten in der wirkstoffhaltigen Schicht nach dem Trocknungsprozeß und gegebenenfalls der damit erfolgenden Abdampfung und/oder unerwünschten Verdunstung von flüchtigen Hilfsstoffen während der Herstellung auftritt. Um vollständige Lösungsmittelfreiheit zu erzielen, die aus Gründen der physikalischen Stabilität und der Hautverträglichkeit des Systems in der Regel anzustreben ist, muß das Reservoir gegebenenfalls in mehreren Schichten aufgebaut werden, was jedoch zu einer Verteuerung der Herstellung führt.

Zur transdermalen Anwendung von Östrogenen und/oder Gestagenen und/oder Androgenen mittels monolithischer Systeme, bei denen der oder die Wirkstoffe in eine selbstklebende Haftklebermatrix eingearbeitet werden, kommen nach dem Stand der Technik - u.a. wegen ihres relativ guten Lösungsvermögens für diese Wirkstoffgruppe - bevorzugt Haftkleber auf Acrylat-Copolymerisatbasis zum Einsatz, ohne (EP 0 416 842, WO 93/10772) oder mit (WO 96/08255, DE 44 05 898) Zusatz von penetrationsfördernden, kristallisationshemmenden (WO 95/09618, WO 93/08795), die Wirkstofflöslichkeit weiter erhöhenden (DE OS 44 05 898) und wasserbindenden (DE 39 33 460) Substanzen.

Die beschriebenen Rezepturen erfordern dabei in der Regel den Einsatz organischer Lösungsmittel, die im Rahmen der Herstellung wieder quantitativ entfernt werden müssen. Auch sind - trotz des relativ einfachen Aufbaus monolithischer TTS - die üblichen pharmazeutischen Qualitätsanforderungen bzgl. Hafteigenschaften, Reproduzierbarkeit der Wirkstofffreisetzung und Lagerstabilität wegen der oben beschriebenen Schwierigkeiten nur mit hohem technischen Aufwand in der Entwicklung und Produktion zu gewährleisten. Häufig müssen großflächige Pflaster appliziert werden, insbesondere zur Verabreichung von Gestagenen, um die therapeutisch erforderlichen Wirkstoffspiegel im Blut über eine mehrtägige Anwendungsdauer aufrechtzuerhalten, wodurch sich zum einen die Gebrauchseigenschaften und damit verbunden die Patientencompliance verschlechtern, zum anderen die Kosten für das Präparat weiter erhöhen.

Ferner sind aus der Literatur monolithische Systeme mit Sexualsteroiden auf Basis von Polystyrolblockcopolymeren als Trägermaterialien bekannt, deren Verwendung prinzipiell eine Fertigung selbstklebender Wirkstoffreservoire aus der Schmelze ohne Einsatz von organischem Lösungsmittel erlaubt. So werden in WO 94/26257 steroidhaltige Haftkleber beschrieben, die Ester des Kolophoniums enthalten und bei denen die Herstellung einer estradiol- und/oder levonorgestrelhaltigen Klebermatrix durch Aufschmelzen und intensives Kneten bei hoher Temperatur über längere Zeiträume erfolgen kann. Transdermale Therapeutische Systeme, die auf diese Weise hergestellt werden, weisen den Nachteil auf, daß sich der oder die Wirkstoffe und/oder pharmazeutische Zusätze unter den Bedingungen des Herstellprozesses teilweise zersetzen, daß die Hafteigenschaften und/oder Hautverträglichkeit des Pflasters über mehrere Tage unzureichend sind, und - insbesondere für die Gestagenkomponente - die erzielbaren Plasmakonzentrationen therapeutisch nicht ausreichen.

Aus der EP 0 186 019 sind ferner Wirkstoffpflaster bekannt, bei denen einer Kautschukklebeharzmasse in Wasser quellbare Polymere zugesetzt sind und aus denen Estradiol freigesetzt werden kann, wobei im Einzelfall ebenfalls eine Herstellung nach dem Hotmelt-Verfahren möglich ist. Auch bei diesen Rezepturen ist es schwierig, ausreichende Mengen an Sexualsteroidhormonen in der Pflastermatrix in Lösung zu halten und über längere Zeiträume in einer annähernd konstanten Rate durch die Haut freizusetzen.

Ferner sind aus der DE 44 29 667 Rezepturen für die transepidermale Abgabe von Estradiol bekannt, die ohne Verwendung von organischen Lösungsmitteln durch Aufschmelzen der Rezepturbestandteile hergestellt werden, wobei als Schutz vor Ausfällung des Estradiol-Hemihydrates bei Lagerung ein Zusatz von Glycerin vorgesehen ist. Die in der Beschreibung bzw. in den Beispielen genannten Kleberformulierungen auf Basis von Polystyrolblockcopolymeren entsprechen dabei dem Stand der Technik, d.h. Wirkstoffaufnahme und -abgabekapazität von TTS dieser Art sind für eine Anwendung des Hormonpflasters über mehrere Tage im allgemeinen, insbesondere im Fall von Gestagenen und Androgenen, zu niedrig.

Neben den monolithischen Systemen sind aus der Literatur auch mehrschichtig aufgebaute Matrix- und Reservoirsysteme hinlänglich bekannt, bei denen Wirkstoffreservoir-, Haftklebeschicht und/oder Freisetzungskontrollschichten funktionell und/oder räumlich voneinander getrennt sind. Die EP 0 285 563 beschreibt ein TTS für die kombinierte Applikation von Östrogenen und Gestagenen. Das Wirkstoffreservoir enthält dabei Ethanol als Lösungs- und Freisetzungssteuerungsmittel für die wirksamen Bestandteile. An der Kontrolle der Steroidhormonfreisetzung ist ferner eine Membran beteiligt, die sich zwischen Reservoir und separat angeordneter Klebeschicht befindet. Die mögliche Anwendungsdauer solcher TTS hängt u.a. stark vom Ethanolgehalt im Reservoir ab (J.A.

Simon et al. (1991), Fertility and Sterility, 56: 1029-1033), der bei der Humananwendung durch Resorption fortlaufend abnimmt und damit die funktionale Lebenszeit des Systems begrenzt. Da neben den Wirkstoffen eine weitere, die Resorption steigernde Komponente in einer relativ hohen Rate freigesetzt wird, besteht je nach Umgebungs-, Lagerungs- und Anwendungsbedingungen das Risiko physikalischer Instabilität, nachlassender Klebkraft und/oder lokaler Hautirritationen.

Ein sog. "enhanced" System, bei dem außer dem Wirkstoff noch Penetrationsbeschleuniger an die Haut freigesetzt werden und welches getrennte Reservoir-, Steuerungs- und Klebeschichten enthält, ist aus dem Stand der Technik ebenfalls für die transepidermale Anwendung von Testosteron (U.S. 5,152,997) bekannt.

Dieses TTS hat für den Patienten den Vorteil, daß es nicht auf die relativ durchlässige Skrotalhaut geklebt werden muß, was wegen sonst zu geringer Wirkstoffabsorption bei Testosteronpflastern ohne Penetrationsvermittler (z.B. gemäß DE OS 35 23 065) der Fall ist. Eine Anwendung solcher "enhanced" Systeme über mehr als 24 Stunden ist jedoch mit einem erhöhten Risiko lokaler Hautreizungen verbunden, bedingt durch die die Hautpermeation von Testosteron steuernden Zusatzstoffe. Es können insbesondere bei ungünstigen Applikationsbedingungen (Schwitzen, starke Hautbewegungen, Duschen) Probleme bzgl. der Hafteigenschaften auftreten.

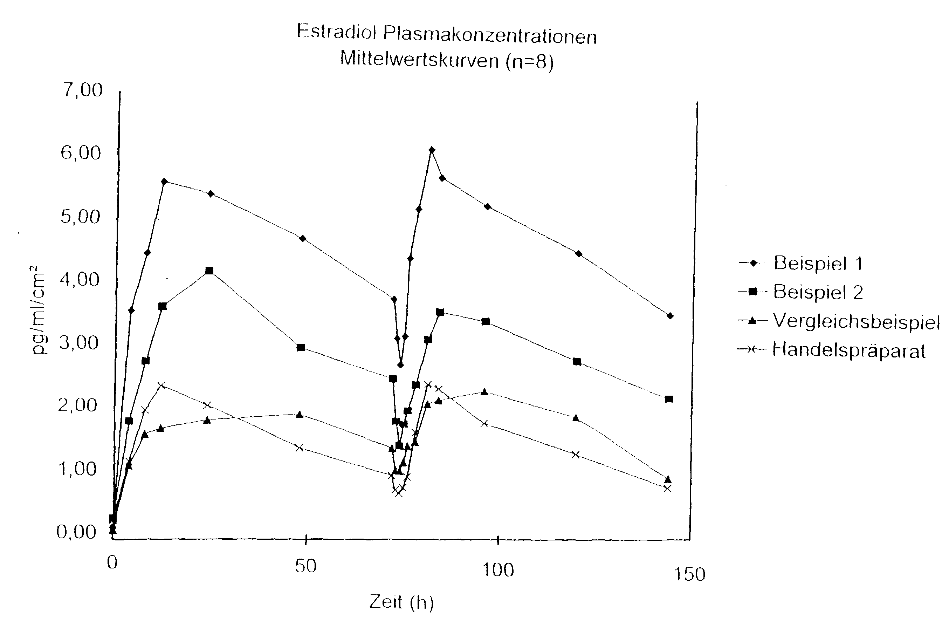

Schließlich sind in der Entwicklung von transdermalen Systemen Polymere auf Acrylsäureester und Methacrylsäureesterbasis von besonderem Interesse wegen ihres relativ guten Aufnahme- und Abgabevermögens für eine Vielzahl von Wirkstoffen. Um die Verwendung von Lösungsmitteln bei der Herstellung von Matrixsystemen auf Poly(meth)acrylatbasis zu vermeiden, ist in **DE 4310012** ein dermales therapeutisches System beschrieben, in dem eine oder mehrere Schichten aus Mischungen von Poly(meth)acrylaten aufgebaut und aus der Schmelze hergestellt werden und die erste Mischungskomponente aus (Meth)acrylpolymeren besteht, die funktionelle Gruppen enthalten, die zweite Mischungskomponente das Fließverhalten reguliert und nur unerhebliche Mengen an funktionellen Gruppen enthält. Die zusammengesetzten Systeme mit Poly(meth)acrylaten mit funktionellen Gruppen sollen eine gesteuerte Abgabe des oder der Wirkstoffe an bzw. durch die Haut und eine einfache Art der Herstellung ermöglichen. Den Vorteilen in der Herstellung gegenüber lösungsmittelbasierten Verfahren stehen bei solchen Systemen jedoch erfahrungsgemäß eine Reihe von Nachteilen gegenüber, die bedingt sind durch:
1. Länger andauernde thermische Belastung aller TTS-Komponenten bei (1) der Herstellung der Polymerschmelze, (2) der homogenen Einarbeitung des oder der Wirkstoffe und/oder (3) der Beschichtung der heißen wirkstoffhaltigen Masse auf geeignete Trägermaterialien mit erhöhtem Risiko von Abbau- bzw. Zersetzungsreaktionen in der Polymerschmelze und/oder während der Lagerung der wirkstoffhaltigen Polymerfilme.
2. Schwierigkeiten in der Optimierung der Co-/Adhäsionsbalance der poly(meth)acrylathaltigen Schicht, da eine Vernetzung des Acrylatcopolymerisates mittels kovalenter Bindungen während der Herstellung der wirkstoffhaltigen Polymermatrix in der Schmelze nicht möglich ist, verbunden mit Problemen, die durch einen kalten Fluß der Polymermasse bei Anwendung auf der Haut und/oder bei Lagerung auftreten können.
3. starke Bindung von 17-β-Estradiol in der Polymermatrix durch Polymethacrylate mit einem hohen Gehalt an freien Aminogruppen, wodurch die Fluxraten des Sexualsteroids gegenüber Polymethacrylat-Matrices ohne freie Aminogruppen - bei gleicher Wirkstoffbeladung - verringert werden (siehe Abb. 1, Vergleichsbeispiel).

Wie die vorgenannte Aufstellung zeigt, sind viele Pflasterkonstruktionen und hierfür verwendete Materialien bekannt. Gleichwohl besteht bis heute für viele in Transdermalen Therapeutischen Systemen verarbeitete Wirkstoffe ein großer Bedarf, TTS zur Verfügung zu stellen, die eine therapeutisch geforderte Wirkstoffabgabe ermöglichen, ohne dabei konstruktiv aufwendig zu sein und in der Gesamtschau ihrer Bestandteile eine optimale Beziehung darstellen.

Dies gilt auch für die Wirkstoffe aus der Gattung der Sexualsteroide, wenn sie transcutan verabreicht werden sollen, insbesondere für Östrogene, Gestagene und Androgene.

Aufgabe der vorliegenden Erfindung ist es daher, die vorgenannten Nachteile der TTS mit Sexualsteroiden zu vermeiden und ein konstruktiv einfaches, hautverträgliches, über längere Lagerungs- und Applikationsdauer physikalisch und chemisch stabiles TTS zur transepidermalen Verabreichung von Sexualsteroiden mit guten Hafteigenschaften zur Verfügung zu stellen, das
a) pro Flächeneinheit möglichst viel Wirkstoff an und durch die Haut freisetzt,
b) lösungsmittelfrei ist
   und
c) bei dem der eingesetzte Wirkstoff bzw. die Wirkstoffe thermisch minimal belastet ist bzw. sind.

Zur Lösung dieser Aufgabe wird ein steroidhormonhaltiges TTS und ein Verfahren zu seiner Herstellung ohne Verwendung von Lösemitteln zur Verfügung gestellt, dessen besondere Zusammensetzung überraschenderweise der vorgenannten Aufgabenstellung genügt.

Das erfindungsgemäße Transdermale Therapeutische System (TTS) enthält eine steroidhormonhaltige Matrixmasse in Form einer Schicht, wobei die Matrixmasse bei bis zu 200°C schmelzextrudierte, ammoniogruppenhaltige (Meth)acrylatcopolymere und mindestens einen Weichmacher alleine oder in Mischung mit einem Ethylenacrylat-Terpolymeren und/oder Polyethyienglykol sowie wenigstens 2 Gew.-% von jedem in der Matrixmasse ungeschmolzen vorhandenen Steroidhormon aufweist und nach außen mit einer Deckschicht versehen ist. Das Laminat aus Deckschicht und steroidhaltiger Matrixmasse ist, mit Ausnahme seiner Freisetzungsfläche auf der Haut, entweder von einem größeren wirkstofffreien Hautpflaster umgeben, das zur Fixierung des TTS an der Applikationsstelle und/oder zum Abdecken der wirkstoffhaltigen Matrix an den offenen Kanten dient, oder auf die Freisetzungsfläche ist hautseitig ein wirkstofffreier Klebefilm aufgetragen. Vorteilhafterweise können als wirkstofffreier Klebefilm verwendet werden: kovalent vernetzbare Acrylatcopolymere oder silikonbasierte Polymere. Die aus den erfindungsgemäßen TTS erzielbaren Freisetzungsraten sind so hoch, daß die Applikationszeit gegenüber aus dem Stand der Technik bekannten Pflastersystemen verlängert werden kann, ohne die Applikationsfläche zu vergrößern (vergl. Abb. 1).

In der Entwicklung des erfindungsgemäßen TTS ist es im Gegensatz zu monolithisch aufgebauten Matrixsystemen vorteilhafterweise möglich, Ko-/Adhäsionseigenschaften des TTS einerseits und Wirkstofflöslichkeit, -lösungsgeschwindigkeit und -freisetzungsverhalten andererseits weitgehend getrennt voneinander zu optimieren. Ferner ist für die erfindungsgemäßen TTS überraschend, daß
(1) bei - gegenüber dem Stand der Technik - hohen Wirkstoffkonzentrationen in der Polymermatrix eine ausreichende physikalische Stabilität des Systems bei Langzeitlagerung gewährleistet ist, und daß
(2) auf die Einbringung von separaten Trennschichten oder Membranen zwischen wirkstoffhaltiger und -freier Schicht verzichtet werden kann.

Überraschenderweise ergänzen sich in den TTS der vorliegenden Erfindung die Hafteigenschaften der wirkstoffhaltigen Matrix und des als Fixierungshilfe vorgesehenen Hautpflasters in der Weise, daß unmittelbar nach dem Aufkleben des TTS ein inniger Kontakt zwischen Wirkstoffmatrix und Haut hergestellt ist, der auch bei Verwendung relativ kleinflächiger Hautpflaster mit einem wirkstofffreien Kleberand von etwa nur 3,5 mm Breite über mehrere Tage erhalten bleibt (Beispiel: Bei insgesamt 20 cm² großen, quadratisch geformten TTS mit abgerundeten Ecken (inkl. Fixierungshilfe) kann somit die Fläche der wirkstoffhaltigen Matrix bis ca. 15 cm² groß sein). Werden gemäß vorliegender Erfindung vernetzbare Haftkleberschichten als Fixierungshilfe direkt auf die Wirkstoffschicht kaschiert, weisen die so erhaltenen selbstklebenden TTS bestehend aus Deckschicht, Wirkstoffschicht und Haftschicht eine über längere Applikationszeiträume ebenfalls überraschend hohe Steroidfreisetzungsrate auf (vergl. Abb. 1, Beispiel 2).

Trotz der hohen Beladung der Matrixmasse mit wenigstens 2 Gew.-% an Wirkstoff(en), kann auf Grund der besonderen qualitativen und quantitativen Zusammensetzung der Matrixmasse keine Rekristallisationsneigung der eingearbeiteten Wirkstoffe beobachtet werden, obwohl gerade die Feuchtigkeitsempfindlichkeit bei hormonhaltigen Pflastern ein großes Problem darstellt. Dadurch kann die aktive Haftfläche des TTS klein gehalten, die funktionelle Lebenszeit des TTS verlängert werden und somit der Patientencompliance Rechnung getragen werden. Überraschenderweise können über 10 Gew-% NETa (= Norethisteronazetat) in die Matrixmasse eingearbeitet werden.

Weiterhin war überraschend, daß in der erfindungsgemäßen wirkstoffhaltigen Matrixzusammensetzung Tributylcitrat als Weichmacher besonders vorteilhaft ist.

Besonders vorteilhaft ist die erfindungsgemäße Ausführungsform, in der die steroidhormonhaltige Matrixmasse eine feste Lösung ist.

Weiterhin kann ein TTS gemäß der Erfindung Östrogene oder Gestagene allein oder in Kombination derselben enthalten.

Schließlich kann ein erfindungsgemäßes TTS in einer Ausführungsform Androgene enthalten.

Vorteilhafterweise weist die für das TTS verwendete Trägerfolie matrixseitig eine Metalldampf- oder Oxidbeschichtung auf.

Im Sinne der Erfindung werden die nachfolgend genannten Begriffe und/oder Worte, wie folgt, verstanden:
- a) "lösemittelfrei":: zur Herstellung der Polymermatrices werden keine Lösungsmittel verwendet, die im Verlaufe des Herstellungsverfahrens wieder weitgehend entfernt werden, wie dieses im sogenannten "solvent based"-Verfahren geschieht,
- b) "längere Applikationszeiträume":: die TTS können zur therapeutischen Anwendung bis zu 7 Tage auf die Haut appliziert werden.
- c) "feste Lösung":: der pharmazeutische Wirkstoff liegt in der Pflastermatrix molekulardispers verteilt vor.
- d) "transepidermal":: ist sinn- und funktionsgleich mit transcutan
- e) "thermisch minimiert belasteter Wirkstoff":: der Wirkstoff wird ungeschmolzen der bei der Schmelzextrusion erhitzten Matrixmasse zugeführt, die nach Wirkstoffzusatz gegekühlt wird.

Das Verfahren zur Herstellung des erfindungsgemäßen TTS ist dadurch gekennzeichnet, daß eine beschichtungsfähige steroidhormonhaltige Matrixmasse durch Schmelzextrusion erzeugt wird, wobei die wirksamen Bestandteile in eine bis zu 200°C heiße Polymerschmelze kontinuierlich eingewogen und nicht vorgeschmolzen eingearbeitet werden, die heiße wirkstoffhaltige Polymerschmelze direkt anschließend auf eine ablösbare Schutzschicht (= Substrat) in einer Dicke von 0,02 bis 0,4 mm beschichtet wird und das erhaltene 2-Schichtlaminat mit einer Deckschicht versehen wird. Zur Fixierung der Wirkstoffmatrix auf der Haut und/oder zum Abdecken der Matrix an den offenen Kanten dient ein größeres wirkstofffreies Hautpflaster oder es wird ein wirkstofffreier Klebefilm aus einem vernetzten Acrylatcopolymeren direkt auf die wirkstoffhaltige Polymermatrix laminiert Die erfindungsgemäßen TTS werden mit einer Schutzfolie versehen, die vor Aufbringen des Präparates auf der Haut entfernt wird.

Ein wesentlicher Vorteil des erfindungsgemäßen Verfahrens besteht darin, daß das Wirkstoffreservoir (I) ohne Verwendung von organischen Lösungsmitteln hergestellt wird, und (II) die Zubereitung der wirkstoffhaltigen Matrixmasse und ihre weitere Verarbeitung zu einer wirkstoffhaltigen Schicht in einem kontinuierlichen und besonders kostensparenden Arbeitsgang erfolgen: Prozeßzeiten lassen sich auf wenige Minuten verkürzen und damit zugleich die Gefahr von Zersetzungsreaktionen in der wirkstoffhaltigen Polymerschmelze auf ein Minimum reduzieren. Überraschend wurde dabei gefunden, daß die vollständige Auflösung des oder der Sexualsteroide in der Polymerschmelze trotz der kurzen Prozeßzeiten unter den in den Beispielen weiter erläuterten Verfahrensbedingungen gewährleistet ist.

Ferner werden durch die kontinuierliche Herstellung der steroidhormonhaltigen Polymermasse Scaling-Up Probleme umgangen. D.h. zur Erhöhung der Ansatz- bzw. Chargengröße ist bei der Herstellung der wirkstoffhaltigen Polymerschmelze und des Laminates kein Wechsel aus größere Produktionsanlagen erforderlich, der üblicherweise mit zeit- und kostenaufwendigen Installations-, Qualifizierungs- und Validierungsarbeiten sowie ggf. auch Rezepturänderungen verbunden ist.

Die Erfindung wird anhand folgender Beispiele erläutert:

### A) TTS, die mit einem Wirkstoff beladen sind

### I. Beispiel 1

Ein mit drei Dosiereinheiten ausgerüsteter Zweischneckenextruder wird kontinuierlich in aufeinanderfolgenden Verfahrenszonen mit Eudragit RS 100 (Copolymerisat aus Ethylacrylat und Methylmethacrylat mit ca. 5 % Trimethylammonioethylmethacrylatchlorid), Tributylcitrat (TBC) und 17β-Estradiol-Hemihydrat beschickt, wobei die Mischung mit einem Gesamtdurchsatz von 150 g Masse/min bei einer Temperatur von 130 - 150°C schmelzextrudiert wird. Aus der Dosiereinheit 1 wird Eudragit RS 100 in einer Rate von 97,2 g/min dem Verfahrensteil des Extruders zugeführt, aus Dosiereinheit 2 Tributylcitrat in einer Rate von 45,82 g/min und schließlich aus Dosiereinheit 3, 17β-Estradiol-Hemihydrat in einer Reihe von 6,975 g/min. Nach dem Verlassen des Extruders wird die erhaltene heiße Estradiol-Polymer-Schmelze über einen beheizten Zuführschlauch in einen kontinuierlichen Strom direkt dem Auftragskopf der Beschichtungsanlage zugeführt und mittels Schlitzdüse auf eine ca. 100 µm dicke silikonisierte Polyesterfolie (= Schutzfolie) so laminiert, daß das Flächengewicht der wirkstoffhaltigen Matrixschicht ca. 80 g pro m² beträgt. Das Zweischichtiaminat wird nach Durchlaufen einer Walzenkühleinrichtung mit einer ca. 20 Mikrometer dicken Polyesterfolie (= Trägerfolie) abgedeckt. Das so erhaltene bahnförmige Dreischichtlaminat wird auf Rollen gewickelt und anschließend in 8 cm² große Stücke ausgestanzt. Die resulitierenden TTS enthalten ca. 2,88 mg 17β-Estradiol, berechnet als Hemihydrat. Die TTS werden zur Applikation auf der Haut nach Entfernen der silikonisierten Polyesterfolie mit einem 16 cm² großen wirkstofffreien Hautpflaster ("Overtape"), bestehend aus einem Haftklebefilm auf Basis eines vernetzten Acrylatcopolymerisates und einer Trägerfolie, überklebt.

### II. Beispiel 2

(1) Herstellung derwirkstoffhaltigen Reservoirschicht
   Die Herstellung der wirkstoffhaltigen Reservoirschicht erfolgt über Schmelzextrusion und Hotmelt-Beschichtung wie unter Beispiel 1 beschrieben.
(2) Herstellung des wirkstofffreien Klebefilms
   750 g einer Lösung eines vernetzbaren Haftklebers auf Acrylatcopolymerbasis mit mindestens einem Derivat der Acryl- und Methacrylsäure (z.B. Duro-Tak 87-2852) werden mit einem Rakelauftragswerk auf eine einseitig mit Aluminium bedampfte und beidseitig mit Silikon beschichtete ca. 100 µm dicke Polyesterfolie ausgestrichen, so daß nach Entfernen des Lösungsmittels bei 40 bis 80°C und einer Trocknungszeit von ca. 18 Min. ein wirkstoffhaltiger Klebefilm mit einem Flächengewicht von 20 g/m² resultiert.
(3) Herstellung der TTS
   Auf den gemäß (2) erhaltenen wirkstofffreien Klebefilm wird die Reservoirschicht aus (1) nach Entfernen der Schutzfolie aufkaschiert und das resultierende 4-schichtig aufgebaute Laminat, bestehend aus Schutzfolie, Haftklebefilm, wirkstoffhaltiger Matrixschicht und Trägerfolie, wird in 10 cm² große Stücke ausgestanzt. Die so hergestellten selbstklebenden TTS enthalten ca. 3,6 mg 17β-Estradiol, berechnet als Hemihydrat.

### III. Vergleichsbeispiel

### (Polymethacrylatmischung in der Zusammensetzung entsprechend DE 43 10 012)

Ein mit vier Dosiereinheiten ausgerüsteter Zweischneckenextruder wird kontinuierlich in aufeinanderfolgenden Verfahrenszonen mit Eudragit RS 100 (Copolymerisat aus Ethylacrylat und Methylmethacrylat mit ca. 5 % Trimethylammoniomethacrylatchlorid), Eudragit E 100 (Copolymerisat aus Butylmethacrylat, Methylmethacrylat und 2-Dimethylaminoethyl-methacrylat mit einem Aminogruppengehalt von 25 %), Tributylcitrat (TBC) und 17β-Estradiolhemihydrat beschickt. Die Mischung wird mit einem Gesamtdurchsatz von 150 g/min bei einer Temperatur von 130 - 150°C schmelzextrudiert. Eudragit RS 100 (1) und Eudragit E 100 (2) werden mittels Dosiereinheit 1 bzw. 2 in einer Rate von (1) 42,75 g/min. bzw. (2) 59,85 g/min in die erste Verfahrenszone des Extruders eingebracht. In zwei nachfolgenden Verfahrenszonen werden TBC mittels Dosiereinheit 3 in einer Rate von 39,9 g/min und Estradiol-Hemihydrat mittels Dosiereinheit 4 in einer Rate von 7,5 g/min zugesetzt. Die Weiterverarbeitung erfolgt entsprechend Beispiel 1, wobei das nach der Beschichtung in Bahnen erhaltene 3-schichtig aufgebaute Laminat, bestehend aus Schutzfolie, wirkstoffhaltiger Matrix mit einem Flächengewicht von 80 g/m² und Trägerfolie, in 16 cm² große Stücke gestanzt wird. Die resultierenden selbstklebenden TTS enthalten ca. 6,4 mg 17β-Estradiol berechnet als Hemihydrat.

### IV. In vitro-Untersuchunqen

### Estradiolfluxmessungen in vitro

Zur Beurteilung der Wirkstofffreisetzung in vitro wird ein TTS mit einer ausgestanzten Fläche von 5 cm² in einer modifizierten Franz-Diffusionszelle auf einer Hautpräparation haarloser Mäuse befestigt. Unmittelbar anschließend wird die Zelle mit destilliertem Wasser als Freisetzungsmedium luftblasenfrei befüllt und auf 37 ± 0,5°C thermostatisiert.

Zu den Probeentnahmezeiten wird das Freisetzungsmedium gegen frisches, auf 37 ± 0,5°C thermostatisiertes Wasser ausgetauscht.

Der Estradiolgehalt in dem jeweils entnommenen Freisetzungsmedium wird mittels Hochdruckflüssigkeitschromatographie bestimmt. Die Ergebnisse der Untersuchungen sind in Tabelle 1 für die Beispiele 1 und 2 sowie Vergleichsbeispiel und einem handelsüblichen Matrixpflaster mit einer deklarierten Freisetzung von 50 µg Estradiol pro Tag dargestellt. Wie die Gegenüberstellung der Fluxraten zeigt, wird aus den erfindungsgemäßen TTS deutlich mehr Estradiol durch die Haut freigesetzt als bei den Vergleichssystemen.

**Tabelle 1:**

| Estradiol-Fluxraten | | | | | |
|---|---|---|---|---|---|
| TTS | Estradiolgehalt (Hemihydrat) mg/cm² | Gew.-% | Kumulative Fluxrate (µg/cm²), Mittelwerte, n=3 nach | | |
| | | | 24 h | 48 h | 72 h |
| Beispiel 1 Beispiel 2 | 0,36 0,36 | 4,65 3,72 | 74,2 33,1 | 131,6 71,0 | 171,1 104,4 |
| Vergleichsbeispiel (DE 43 10 012) | 0,40 | 5,00 | 24,0 | 48,0 | 70,5 |
| Handelspräparat (Matrixpflaster) | 0,20 | 2,50 | 22,9 | 38,8 | 51,8 |

### V. Bioverfügbarkeit von Estradiol aus den erfindungsgemäßen TTS

In einer offenen humanpharmakologischen Studie wurde die Estradiol-Bioverfügbarkeit an 8 postmenopausalen Frauen in einem 4-fach cross-over Studiendesign mit den in Tabelle 1 genannten TTS geprüft. Jeder TTS-Typ wurde über einen Zeitraum von 6 Tagen mit wiederholter Gabe, d.h. Pflasterwechsel nach 3 Tagen, appliziert. Entsprechend der Anzahl von Vergleichs- und Testpräparaten wurden insgesamt 4 Behandlungen mit je 2 unmittelbar aufeinanderfolgenden Pflasterapplikationen durchgeführt.

Die Auswaschphase zwischen 2 Behandlungen betrug mindestens 1 Woche. Um die transepidermal resorbierten Wirkstoffmengen zu bestimmen, wurde den Probandinnen in festgelegten Zeitabständen Blut entnommen. Zur quantitativen Bestimmung der Estradiolkonzentration im Blutplasma diente eine empfindliche GC/MS-Methode (Quantifizierungsgrenze: 5pg/ml).

Der zeitliche, flächennormierte Verlauf der erhaltenen Estradiolplasmakonzentrationen über 6 Tage (jeweils mit TTS-Wechsel nach 3 Tagen) ist in Abbildung 1 für Test- und Vergleichspflaster dargestellt. Wie die Abbildung zeigt, wurden mit den erfindungsgemäßen TTS pro Flächeneinheit deutlich höhere Estradiolspiegel im Plasma erzielt i. Vgl. zu TTS entsprechend Vergleichsbeispiel und dem geprüften Handelspräparat.

### B) TTS, die mit mehr als einem Wirkstoff beladen sind

### Beispiel 3

Ein mit vier Dosiereinheiten ausgerüsteter Zweischneckenextruder wird kontinuierlich in aufeinanderfolgenden Verfahrenszonen mit Eudragit RS 100 (Copolymerisat aus Ethylacrylat und Methylmethacrylat mit ca. 5 % Trimethylammonioethylmethacrylatchlorid), Tributylcitrat (TBC), 17β-Estradiol-Hemihydrat und Norethisteronazetat beschickt, wobei die Mischung mit einem Gesamtdurchsatz von 116,7 g Masse/min bei einer Temperatur von 130-150°C schmelzextrudiert wird. Aus der Dosiereinheit 1 wird Eudragit RS 100 in einer Rate von 62,24 g/min dem Verfahrensteil des Extruders zugeführt, aus Dosiereinheit 2 Tributylcitrat in einer Rate von 29,34 g/min, aus Dosierstation 3 17β-Estradiol-Hemihydrat in einer Rate von 4,667 g/min und schließlich aus Dosierstation 4 Norethisteronazetat in einer Rate von 20,417 g/min. Nach dem Verlassen des Extruders wird die erhaltene heiße Estradiol-Norethisteronazetat-Polymer-Schmelze über einen beheizbaren Zuführschlauch direkt in einen kontinuierlichen Strom, dem Auftragskopf der Beschichtungsanlage zugeführt und mittels Schlitzdüse auf eine ca. 100 µm dicke silikonisierte Polyesterfolie (= Schutzfolie) so laminiert, daß das Flächengewicht der Reservoirschicht ca. 80 g pro µm beträgt. Das Zweischichtlaminat wird nach Durchlaufen einer Walzenkühleinrichtung mit einer ca. 20 Mikrometer dicken Polyesterfolie (= Trägerfolie) abgedeckt. Das so erhaltene bahnförmige Dreischichtlaminat wird auf Rollen gewickelt und anschließend in 16 cm² große Stücke ausgestanzt. Die resultierenden TTS-Reservoire enthalten ca. 5,12 mg 17β-Estradiol, berechnet als Hemihydrat und ca. 22,40 mg Norethisteronazetat. Die TTS werden zur Applikation auf der Haut nach Entfernen der silikonisierten Polyesterfolie mit einem 32 cm² großen wirkstofffreien Hautpflaster ("Overtape"), bestehend aus einem Haftklebefilm auf Basis eines vernetzten Acrylatcopolymerisates und einer Trägerfolie, überklebt.

**Tabelle 4**

| Fluxraten von Estradiol (E2)- und Norethisteronazetat (NETa) durch exzidierte Mäuse-haut | | | | | |
|---|---|---|---|---|---|
| Beispiel 6 | Wirkstoffgehalt | | Kumulative Fluxraten µg/cm², Mittelwerte, n=3 | | |
| | mg/cm² | Gew.-% | nach 24 h | nach 48 h | nach 72 h |
| E2 (Hemihydrat) | 0,3 | 4,0 | 16,0 | 33,2 | 48,9 |
| NETa | 1,4 | 17,5 | 34,3 | 73,9 | 114,5 |

### In vitro-Untersuchungen

Die Bestimmung erfolgt wie unter II. beschrieben. Die gemessenen Fluxraten sind in Tabelle 4 wiedergegeben, zusammen mit dem Wirkstoffgehalt der Prüfmuster. Wie der Tabelle zu entnehmen ist, lassen sich nach dem erfindungsgemäßen Verfahren Kombination TTS mit einem hohen NETa-Gehalt in einer hohen Fluxrate durch exzidierte Hautpräparationen herstellen.

## Patentansprüche

1. Transdermales Therapeutisches System (TTS) zur transcutanen Verabreichung von Sexualsteroiden über längere Zeiträume mit einer Fixierungshilfe für das TTS auf der Haut, **dadurch gekennzeichnet, daß** das TTS eine schichtförmige steroidhormonhaltige Matrixmasse enthält, die ammonio-gruppenhaltige (Meth)acrylatcopolymere und mindestens einen Weichmacher alleine oder in Mischung mit einem Ethylenacrylat-Terpolymeren, wenigstens 2 Gewichtsprozent von jedem in der Matrixmasse molekulardispers verteilten Steroidhormon enthält und dieses, mit Ausnahme seiner Freisetzungsfläche auf der Haut, von einem größeren wirkstofffreien Pflaster zur Fixierung an der Applikationsstelle umgeben ist.

2. TTS nach Anspruch 1, **dadurch gekennzeichnet, daß** kantseitig auf die steroidhormonhaltige Matrixmasse ein wirkstofffreier Klebefilm, bestehend aus vernetzten Acrylatcopolymeren aufgetragen ist, der die wirkstoffhaltige Matrix auf der Haut fixiert.

3. TTS nach Ansprüchen 1 oder 2, **dadurch gekennzeichnet, daß** es mindestens einen thermisch minimiert belasteten Wirkstoff enthält.

4. TTS nach Ansprüchen 1 bis 3, **dadurch gekennzeichnet, daß** die steroidhormonhaltige Matrixmasse eine feste Lösung ist.

5. TTS nach Ansprüchen 1 bis 4, **dadurch gekennzeichnet, daß** die steroidhormonhaltige Matrixmasse mindestens einen Weichmacher enthält.

6. TTS nach Ansprüchen 1 bis 5, dadurch gekennzeichent, daß die steroidhormonhaltige Matrix als Weichmacher Zitronensäuretriester enthält.

7. TTS nach Ansprüchen 1 bis 6, **dadurch gekennzeichnet, daß** es Östrogene oder Gestagene alleine oder in Kombination derselben enthält.

8. TTS nach Ansprüchen 1 bis 7, **dadurch gekennzeichnet, daß** es Androgene enthält.

9. TTS nach Ansprüchen 1 bis 8, **dadurch gekennzeichnet, daß** die Trägerfolie matrixseitig eine Metalldampf- oder Oxidbeschichtung aufweist.

10. Verfahren zur Herstellung eines Transdermalen Therapeutischen Systems (TTS) nach Ansprüchen 1 und 3 bis 9, **dadurch gekennzeichnet, daß** (1) eine homogene, beschichtungsfähige steroidhormonhaltige Matrixmasse durch Schmelzextrusion erzeugt wird, indem in einer bis zu 200°C heißen Polymerschmelze, bestehend aus einem ammoniogruppenhaltigen Methacrylatcopolymeren und mindestens einem Weichmacher alleine oder in Mischung mit einem Ethylenacrylat-Terpolymeren, wenigstens bis zu 2 Gewichtsprozent eines jeden Steroidhormons kontinuierlich eingewogen und ungeschmolzen eingearbeitet werden, (2) kontinuierlich ein Träger mit der nach (1) erzeugten heißen wirkstoffhaltigen Polymerschmelze in einer Dicke von 0,02 bis 0,4 mm beschichtet wird, (3) das gemäß (2) erhaltene 2-Schichtlaminat mit einer Deckschicht versehen wird und (4) hierauf ein größeres wirkstofffreies Pflaster zur Fixierung des TTS auf der Haut aufgebracht wird.

11. Verfahren zur Herstellung eines TTS nach Ansprüchen 2 und 3 bis 9, **dadurch gekennzeichnet, daß** eine homogene, beschichtungsfähige steroidhormonhaltige Matrixmasse durch Schmelzextrusion gebildet wird, indem in einer bis zu 200°C heißen Polymerschmelze, bestehend aus einem ammoniogruppenhaltigen Methacrylatcopolymeren und mindestens einem Weichmacher, alleine oder in Mischung mit einem Ethylenacrylat-Terpolymeren, wenigstens bis zu 2 Gewichtsprozent eines jeden Steroidhormons kontinuierlich eingewogen und ungeschmolzen eingearbeitet werden, kontinuierlich ein geeigneter Träger mit der heißen wirkstoffhaltigen Polymerschmelze in einer Dicke von 0,02 bis 0,4 mm beschichtet wird und hierauf ein wirkstofffreier Klebefilm, bestehend aus vernetztem Acrylat-Copolymeren zur Fixierung an der Haut aufgebracht wird.

## Claims

1. Transdermal Therapeutic System (TTS) for transcutaneal administration of sexual steroids over long periods, comprising a fixing aid for the TTS on the skin, **characterised in that** the TTS contains a layered steroid hormone containing matrix substance which contains ammonium group containing (meth) acrylate copolymers and at least one softener on its own or in a mixture with an ethylene acrylate terpolymer, at least 2 weight-% of each steroid hormone molecularly dispersed in the matrix substance, and this is, with the exception of its release surface on the skin, surrounded by a larger active substance free plaster for fixing to the point of application.

2. TTS according to Claim 1, **characterised in that** at the side of the edge is applied to the steroid hormone containing matrix substance an active substance free adhesive film which consists of cross-linked acrylate copolymers which fixes the active substance containing matrix to the skin.

3. ITS according to Claims 1 or 2, **characterised in that** it contains at least one active substance the thermal load of which is minimised.

4. TTS according to Claims 1 to 3, **characterised in that** the steroid hormone containing matrix substance is a solid solution.

5. TTS according to Claims 1 to 4, **characterised in that** the steroid hormone containing matrix substance contains at least one softener.

6. TTS according to Claims 1 to 5, **characterised in that** the steroid hormone containing matrix contains citric acid triester as softener.

7. TTS according to Claims 1 to 6, **characterised in that** it contains ostrogens or gestagens on their own or in a combination thereof.

8. TTS according to Claims 1 to 7, **characterised in that** it contains androgens.

9. TTS according to Claims 1 to 8, **characterised in that** the support foil has at the side of the matrix a metal vapour or oxide coating.

10. Method of producing a transdermal therapeutic system (TTS) according to Claims 1 and 3 to 9, **characterised in that** (1) a homogenous coatable steroid hormone containing matrix substance is produced by way of melt extrusion **in that** into a polymer melt heated up to 200°C and consisting of an ammonium group containing methacrylate copolymer and at least one softener on its own or in a mixture with an ethylene acrylate terpolymer, at least up to 2 weight-% of each steroid hormone is continuously weighed in and processed in an unmolten state; (2) a carrier is continuously coated at a thickness of between 0.02 and 0.4 mm with an active substance containing polymer melt produced according to (1); (3) the 2-layer laminate obtained according to (2) is provided with a covering layer; and (4) thereafter a larger active substance free plaster is attached to the skin for the purpose of fixing the TTS.

11. Method of producing a TTS according to Claims 2 and 3 to 9, **characterised in that** a homogenous coatable steroid hormone containing matrix substance is formed by way of melt extrusion **in that** into a polymer melt, which is heated up to 200°C and which is composed of an ammonium group containing methacrylate copolymer and at least one softener, is continuously weighed on its own or in a mixture with an ethylenacrylate terpolymer and worked thereinto in an unmolten state at least up to 2 weight-% of each steroid hormone, and a suitable carrier is continuously coated with the hot effective polymer melt at a thickness of between 0.02 and 0.4 mm, whereafter a non effective adhesive film of crosslinked copolymers is applied for the purpose of fixing to the skin.

## Revendications

1. Système thérapeutique transdermique (STT) servant à l'administration transcutanée d'hormones stéroïdes sexuelles sur des périodes prolongées avec une aide à la fixation pour le STT sur la peau, ***caractérisé en ce que*** le STT contient une masse matricielle en forme de couche contenant des hormones stéroïdiennes, qui contient des copolymères de (méth)acrylate contenant des groupes ammonio et au moins un plastifiant seul ou en mélange avec un terpolymère d'éthylène-acrylate, au moins 2 % en poids de chaque hormone stéroïdienne dispersée moléculairement dans la masse matricielle, et celle-ci est entourée, à l'exception de sa surface de libération sur la peau, d'un plus grand pansement dépourvu de substance active pour la fixation sur la zone d'application.

2. STT selon la Revendication 1, ***caractérisé en ce que,*** du côté du bord, sur la masse matricielle contenant des hormones stéroïdiennes est fixé un film adhésif dépourvu de substance active, composé de copolymères d'acrylate réticulés, qui fixe la matrice contenant des substances actives sur la peau.

3. STT selon les Revendications 1 ou 2, ***caractérisé en ce qu***'il contient au moins une substance active à charge thermique minimale.

4. STT selon les Revendications 1 à 3, ***caractérisé en ce que*** la masse matricielle contenant des hormones stéroïdiennes est une solution solide.

5. STT selon les Revendications 1 à 4, ***caractérisé en ce que*** la masse matricielle contenant des hormones stéroïdiennes contient au moins un plastifiant.

6. STT selon les Revendications 1 à 5, ***caractérisé en ce que*** la masse matricielle contenant des hormones stéroïdiennes contient comme plastifiant du triester de l'acide citrique.

7. STT selon les Revendications 1 à 6, ***caractérisé en ce qu'***il contient des oestrogènes ou des progestatifs seuls ou en combinaison.

8. STT selon les Revendications 1 à 7, ***caractérisé en ce qu***'il contient des androgènes.

9. STT selon les Revendications 1 à 8, ***caractérisé en ce que*** la feuille support présente côté matrice un revêtement de vapeur métallique ou d'oxyde.

10. Procédé de fabrication d'un système thérapeutique transdermique (STT) selon les Revendications 1 et 3 à 9, ***caractérisé en ce que*** (1) une masse matricielle homogène contenant des hormones stéroïdiennes et apte au revêtement est produite par extrusion de matière fondue, dans lequel, dans une masse fondue de polymères chaude à une température allant jusqu'à 200 °C, composée d'un copolymère de méthacrylate contenant des groupes ammonio et au moins un plastifiant seul ou en mélange avec un terpolymère d'éthylène-acrylate, au moins 2 % en poids de chaque hormone stéroïdienne sont pesés et introduits à l'état non fondu, (2) un support est enduit de manière continue avec la masse fondue de polymères chaude contenant des substances actives produite en (1) sur une épaisseur de 0,02 à 0,4 mm, (3) le stratifié à 2 couches obtenu en (2) est muni d'une couche de couverture et (4) un pansement plus grand dépourvu de substance active est appliqué pour fixer le STT sur la peau.

11. Procédé de fabrication d'un système thérapeutique transdermique (STT) selon les Revendications 2 et 3 à 9, ***caractérisé en ce qu***'une masse matricielle homogène contenant des hormones stéroïdiennes et apte au revêtement est produite par extrusion de matière fondue, dans lequel, dans une masse fondue de polymères chaude à une température allant jusqu'à 200 °C, composée d'un copolymère de méthacrylate contenant des groupes ammonio et au moins un plastifiant seul ou en mélange avec un terpolymère d'éthylène-acrylate, au moins 2 % en poids de chaque hormone stéroïdienne sont pesés et introduits à l'état non fondu, un support adapté est enduit de manière continue avec la masse fondue de polymères chaude contenant des substances actives sur une épaisseur de 0,02 à 0,4 mm, et un film adhésif dépourvu de substance active, composé de copolymères d'acrylate réticulés, est appliqué par-dessus pour la fixation sur la peau.
